(19) Europäisches Patentamt
European Patent Office
Office européen des brevets

(11) Veröffentlichungsnummer: **0 246 196**
**B1**

# (12) EUROPÄISCHE PATENTSCHRIFT

(45) Veröffentlichungstag der Patentschrift: **25.10.89**

(51) Int. Cl.⁴: **C07F 15/00**, C07C 29/136

(21) Anmeldenummer: **87810296.1**

(22) Anmeldetag: **11.05.87**

(54) **Optisch aktive Iridiumkomplexe, Verfahren zu deren Herstellung und deren Verwendung.**

(30) Priorität: **16.05.86 CH 1986/86**

(43) Veröffentlichungstag der Anmeldung: **19.11.87 Patentblatt 87/47**

(45) Bekanntmachung des Hinweises auf die Patenterteilung: **25.10.89 Patentblatt 89/43**

(84) Benannte Vertragsstaaten: **BE CH DE FR GB IT LI NL SE**

(56) Entgegenhaltungen:
**JOURNAL OF ORGANOMETALLIC CHEMISTRY, Band 222, 1981, Seiten 323-329, Elsevier Sequoia S.A., Lausanne, CH; G. ZASSINOVICH et al.: "Enantioselective transfer hydrogenation catalyzed by iridium(I) complexes with nitrogen donor ligands"**

(73) Patentinhaber: **CIBA-GEIGY AG, Klybeckstrasse 141, CH-4002 Basel(CH)**

(72) Erfinder: **Kaschig, Jürgen, Dr., Rötebuckweg 30, D-7800 Freiburg(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft optisch aktive kationische Iridium (I)-Komplexe mit einem asymmetrischen Sekundäraminliganden und einem Dienliganden, ein Verfahren zu deren Herstellung und deren Verwendung als homogene enantioselektive Katalysatoren.

G. Zassinovich et al beschrieben im Journal of Organometallic Chemistry, 222, S. 323 - 329 (1981) kationische Iridium (I)-Komplexe mit einem 1,5-Cyclooctadienliganden und einem 2-Pyridinaldiminliganden der am Imin-N-Atom durch optisch aktives α-Phenylethyl oder 3-Pinanmethyl substituiert ist. Sie wirken als enantioselektive homogene Katalysatoren bei der Transferhydrierung von prochiralen Ketonen mit Isopropanol. Bei der Reaktion werden zwar hohe Ausbeuten erzielt, aber die optische Ausbeute (Enantiomerenüberschuss) ist relativ gering.

H. Brunner et al. beschreiben in Chem. Ber., 117, S. 1330 - 1354 (1984) kationische Rhodium(I)-Komplexe mit einem Cycloocta-1,5-dienliganden und einem asymmetrischen α-(Sekundäraminomethyl)pyridinliganden als homogene Katalysatoren für die enantionselektive Hydrosilyrierung von prochiralen Ketonen.

Gegenstand der Erfindung sind optisch aktive Iridiumkomplexe der Formel I und Gemische der Diastereomeren,

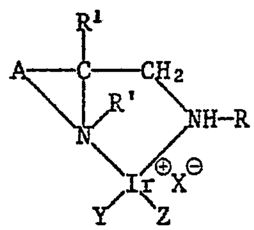 (I),

worin $R^1$ und R′ H bedeuten und A für einen Rest der Formeln II, IIa oder IIb

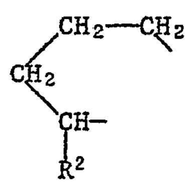 (II), 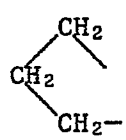 (IIa), 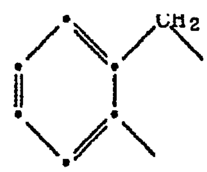 (IIb)

steht, oder $R^1$ und R′ eine Bindung und A einen Rest der Formel IIc

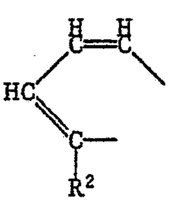 (IIc)

darstellen, in denen $R^2$ -H oder $-CH_3$ ist, $X^{\ominus}$ das Anion einer anorganischen oder organischen Säure darstellt, Y und Z je Ethylen oder Y und Z zusammen ein offenkettiges oder cyclisches Dien mit 6 - 10 C-Atomen, dessen Diengruppen über ein oder zwei C-Atome verbunden sind, bedeuten; und R ein Kohlenwasserstoffrest mit mindestens einem chiralen C-Atom oder ein mindestens ein Heteroatom enthaltender Kohlenwasserstoffrest mit mindestens einem chiralen C-Atom ist, oder A einen Rest der Formeln II, IIa oder IIb und R Phenyl, Naphthyl, 2-Methylphen-1-yl oder 2,6-Dimethylphen-1-yl bedeuten.

Optisch aktiv bedeutet, dass mindestens ein chirales C-Atom überwiegend S- oder R-Konfiguration besitzt.

Bei $X^{\ominus}$ als Anion einer einbasischen anorganischen oder organischen Säure kann es sich zum Beispiel um $F^{\ominus}$, $Cl^{\ominus}$, $Br^{\ominus}$, $J^{\ominus}$, $ClO_4^{\ominus}$, $NO_3^{\ominus}$, $BrO_3^{\ominus}$, $HSO_4^{\ominus}$, $H_2PO_3^{\ominus}$, $H_2PO_4^{\ominus}$, $BF_4^{\ominus}$, $B(Phenyl)_4^{\ominus}$, $PF_6^{\ominus}$, $SbF_6^{\ominus}$, $AsF_6^{\ominus}$, $SbCl_6^{\ominus}$, $SbCl_5F^{\ominus}$, $HCOO^{\ominus}$, $CH_3COO^{\ominus}$, $CCl_3COO^{\ominus}$, $CF_3COO^{\ominus}$, $CH_3SO_3^{\ominus}$, $CCl_3SO_3^{\ominus}$, $CF_3SO_3^{\ominus}$, Phenyl-$SO_3^{\ominus}$ oder p-Toluyl-$SO_3^{\ominus}$ handelt. In einer bevorzugten Ausführungform stellt $X^{\ominus}$ $BF_4^{\ominus}$, $ClO_4^{\ominus}$, $CF_3SO_3^{\ominus}$, $PF_6^{\ominus}$ dar.

Y und Z stehen bevorzugt je für Ethylen oder Y und Z stellen zusammen bevorzugt ein Dien mit 6 bis 8 C-Atomen, dessen Diengruppen insbesondere über 2 C-Atome verbunden sind, dar. In einer bevorzugten Ausführungsform stehen Y und Z zusammen für 1,5-Cyclooctadien, Norbornadien oder 1,5-Hexadien.

Eine bevorzugte Untergruppe der Iridiumkomplexe sind solche der Formel I, worin R ein Rest der Formel III

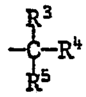 (III)

ist, worin $R^3$, $R^4$ und $R^5$ voneinander verschieden sind, wenn sie nicht mindestens 1 chirales C-Atom enthalten, und für ein Wasserstoffatom, $C_1$-$C_4$-Alkyl, $C_1$-$C_4$-Alkoxy, unsubstituiertes oder durch $C_1$-$C_4$-Alkyl oder Phenyl substituiertes Cycloalkyl mit 5 bis 7 Ring-C-Atomen, unsubstituiertes oder durch $C_1$-$C_4$-Alkyl oder Phenyl substituiertes Cycloalkylalkyl mit 5 bis 7 Ring-C-Atomen und 1 oder 2 C-Atomen in der Alkylengruppe, Phenyl, Naphthyl, Benzyl oder β-Phenylethyl sind; oder $R^4$ und $R^5$ zusammen durch $C_1$-$C_4$-Alkyl oder Phenyl substituiertes lineares $C_4$- oder $C_5$-Alkylen, $C_3$- oder $C_4$-Oxaalkylen oder $C_3$-Dioxaalkylen mit mindestens einem chiralen C-Atom bedeuten. In einer bevorzugten Ausführungsform stellt $R^3$ in Formel III ein Wasserstoffatom dar.

Bei den $C_1$-$C_4$-Alkyl- und $C_1$-$C_4$-Alkoxyresten kann es sich um Methyl, Ethyl, n-Propyl, i-Propyl, n-Butyl, i-Butyl, t-Butyl und die entsprechenden Alkoxyreste handeln. $R^3$, $R^4$ und $R^5$ sind als Alkyl bevorzugt Methyl oder Ethyl und als Alkoxy bevorzugt Methoxy. $R^3$ bis $R^5$ bedeuten als Cycloalkyl bevorzugt Cyclopentyl, Cycloheptyl und besonders Cyclohexyl. Bei $R^2$ bis $R^4$ als Cycloalkylalkyl handelt es sich bevorzugt um Cyclohexylmethyl. $R^3$ und $R^4$ zusammen bedeuten als $C_3$- oder $C_4$-Oxaalkylen bevorzugt 2-Oxabutylen oder 2- oder 3-Oxapentylen und als $C_3$-Dioxaalkylen bevorzugt 2,4-Dioxapentylen. $C_1$-$C_4$-Alkyl als Substituent für $R^3$ bis $R^5$ kann Methyl, Ethyl, n-Propyl, i-Propyl und Butyl sein. Bevorzugte Substituenten sind Methyl und Phenyl.

Eine bevorzugte Untergruppe der Iridiumkomplexe der Formel I sind solche, worin in Formel III $R^3$ H bedeutet, $R^4$ für $C_1$-$C_4$-Alkyl, $C_1$-$C_4$-Alkoxy oder Phenyl und $R^5$ für Phenyl, Benzyl oder Naphthyl stehen, wobei $R^3$ und $R^4$ nicht beide Phenyl sind; oder $R^3$ und $R^5$ je H bedeuten und $R^4$ der Formel

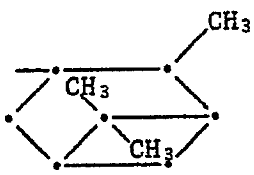

entspricht; oder die Gruppe $-CR^3R^4R^5$ der Formel

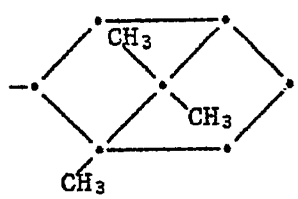

entspricht; oder $R^3$ für H steht und $R^4$ und $R^5$ zusammen in 2-Stellung durch $C_1$-$C_4$-Alkyl substituiertes Pentamethylen oder in 1- und/oder 3-Stellung durch $C_1$-$C_4$-Alkyl oder Phenyl substituiertes 2,4-Dioxapentylen bedeuten.

Eine weitere Ausführungsform bevorzugter Iridiumkomplexe sind solche, worin der Rest der Formel III den Resten

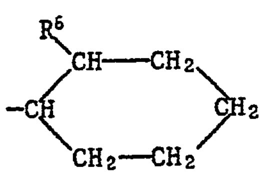 oder 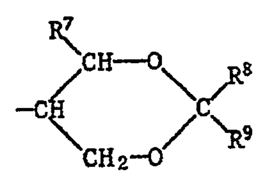

entspricht, worin $R^6$ Methyl oder Phenyl ist, $R^7$ Methyl oder Phenyl darstellen, $R^8$ und $R^9$ Methyl oder $R^8$ H und $R^9$ Phenyl bedeuten.

Insbesondere bevorzugt sind Iridiumkomplexe der Formel I, worin $R^1$ und $R'$ eine Bindung und A ein

Rest der Formel IIc ist, in dem $R^2$ für Methyl steht, und R den Resten

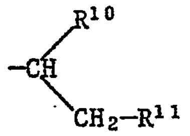

oder

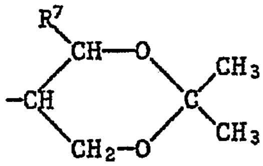

entspricht, worin $R^7$ Phenyl ist, $R^{10}$ Phenyl oder Naphthyl und $R^{11}$ H oder $R^{10}$ und $R^{11}$ Phenyl sind.

Eine weitere bevorzugte Untergruppe sind Iridiumkomplexe der Formel I, worin $R^1$ und R′ eine Bindung und A ein Rest der Formel IIC sind, indem $R^2$ für H steht, und R 2-Methylcyclohex-1-yl, 2-Phenyl-cyclohex-1-yl,

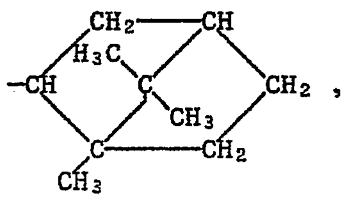

,

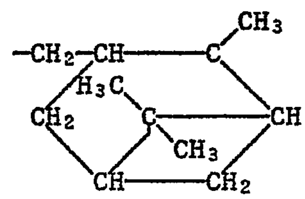

oder

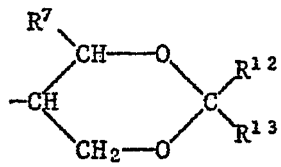

ist,

worin $R^7$ Phenyl und $R^{12}$ und $R^{13}$ $-CH_3$ oder $R^{12}$ H und $R^{13}$ Phenyl bedeuten.

In einer bevorzugten Ausführungsform sind die Iridiumkomplexe der Formel I solche, worin $R^1$ und R′ H und A ein Rest der Formel II sind und R den Rest

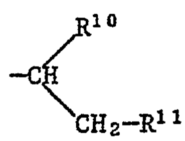

darstellt, worin $R^{10}$ und $R^{11}$ Phenyl, oder $R^{10}$ Phenyl oder Naphthyl und $R^{11}$ H oder $R^{10}$ Methyl und $R^{11}$ Phenyl bedeuten.

Von den Iridiumkomplexen der Formel I, worin $R^1$ und R′ je H und A ein Rest der Formel IIa sind, sind solche bevorzugt, in denen R für Phenyl, 2-Methylphen-1-yl oder 2,6-Dimethylphen-1-yl steht.

Es sind ferner Iridiumkomplexe der Formel I bevorzugt, in denen $X^{\ominus}$ $BF_4^{\ominus}$ und Y und Z zusammen für 1,5-Cyclooctadien stehen.

Die Iridiumkomplexe der Formel I können nach an sich bekannten Verfahren [siehe Inorganica Chimica Acta 73 (1983), S. 275 - 279] erhalten werden, indem man $[(Acetonitril)_2(YZ)]IrX$ mit einem optisch aktiven sekundären Amin der Formel IV

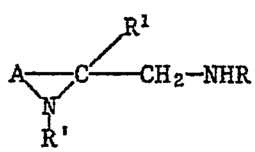

(IV)

umsetzt, worin Y, Z, X, A, R, $R^1$ und R′ die in Anspruch 1 angegebene Bedeutung haben. Die Herstellung des Acetonitrilkomplexes ist dort ebenfalls beschrieben. Die zur Herstellung des Acetonitrilkomplex verwendeten Komplexe $[IrCl(YZ)]_2$ sind z.B. durch die Umsetzung von Dichlorotetrakis(alken)diiridium(I) (Alken z.B. Cycloocten) mit Ethylen oder einem Dien YZ erhältlich.

Die Umsetzungen werden im allgemeinen bei Temperaturen von -10 bis 30°C in einem inerten Lösungsmittel und unter Sauerstoff- und Feuchtigkeitsausschluss durchgeführt. Geeignete inerte Lösungsmittel sind. z.B. Kohlenwasserstoffe wie Benzol, Toluol, Xylol, Petrolether, Hexan, Cyclohexan, Methylcyclohexan; und Ether wie z.B. Diethylether, Dibutylether, Tetrahydrofuran und Dioxan sowie halogenierte Kohlenwasserstoffe, z.B. Chloroform, Methylenchlorid und Chlorbenzol. Zur Herstellung von Salzen der Formel I mit Anionen einbasischer anorganischer oder organischer Säuren können die Salze der Formel I entweder direkt nach der Reaktion oder nach der Isolierung und Reinigung und dem erneuten Lösen in polaren Lösungsmitteln (z.B. Alkoholen, Ethern oder Ketonen, gegebenenfalls unter Zusatz von Wasser) mit einem Alkalisalz $M^{\oplus}X'^{\ominus}$ umgesetzt und danach isoliert werden. $X'^{\ominus}$ bedeutet ein von $X^{\ominus}$ verschiedenes Anion einer einbasischen anorganischen oder organischen Säure, $M^{\oplus}$ steht bevorzugt für Natrium. Die erfindungsgemässen Iridiumkomplexe sind kristallin und können durch Filtration isoliert und durch Umkristallisation gereinigt werden. Optisch aktive sekundäre Amine der Formel IV sind teilweise bekannt oder käuflich, oder können nach bekannten Verfahren hergestellt werden. Amine der Formel IV, worin A ein Rest der Formel IIc ist und $R^1$ und R' eine Bindung darstellen, werden in einfacher Weise durch katalytische Hydrierung der entsprechenden Imine erhalten, z.B.

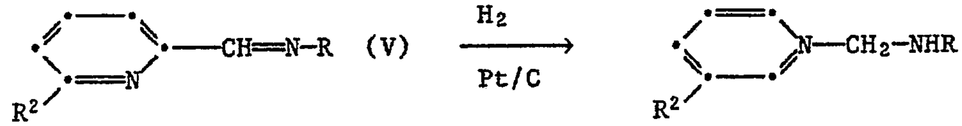


Die Pyridinaldimine der Formel V sind bekannt oder können in an sich bekannter Weise durch die Umsetzung des gegebenenfalls durch Methyl substituierten 2-Pyridinaldehyds mit einem Amin $RNH_2$ erhalten werden. Zweckmässig werden reine Stereoisomere der Amine $RNH_2$ verwendet, so dass man direkt zu reinen Stereoisomeren der Formel V gelangt. Man kann aber auch Racemate einsetzen und anschliessend die gebildeten Racemate der Formel V nach klassischen Methoden auftrennen.

Stereoisomere der Amine $RNH_2$ sind bekannt, teilweise käuflich oder nach bekannten Verfahren herstellbar. Beispiele für solche Amine sind:
(R)-2-Aminobutan, (R)-1-Phenylethylamin, (S)-1-Phenylethylamin, (R)-1-(α-Naphthylethyl)amin, (S)-2-Amino-3-phenylpropan, (R)-1,2-Diphenylethylamin, (S)-Alaninol, (S)-Phenylalaninol, (4S, 5S)-5- Amino-2,2-dimethyl-4-phenyl-1,3-dioxan, (S)-2-Amino-1-methoxy-3-phenyl-propan, (R)-Bornylamin, (R)-3-Aminomethylpinan, (+)-Dehydroabiethylamin, (2R, 4S, 5S)-(+)-5-Amino-2,4-Diphenyl-1,3-dioxan [Chem. Ber. 113, S. 710 - 721 (1980)], (1S, 2R)-(-)-2-Methylcyclohexylamin [Chem. Ber. 117, S.2076 - 2098 (1984)], (1S, 2S)-(+)-2-Phenylcyclohexylamin [Chem. Ber. 117, S. 2076 - 2098 (1984)].

Optisch aktive sekundäre Amine der Formel IV, worin A ein Rest der Formel IIa ist und $R^1$ und R' für H stehen, können ebenfalls durch katalytische Hydrierung mit z.B. $PtO_2$ als Katalysator aus den Iminen der Formel V erhalten werden.

Optisch aktive sekundäre Amine der Formel IV, worin A ein Rest der Formeln II, IIa oder IIb ist und $R^1$ und R' für H stehen, können nach folgendem Verfahren hergestellt werden (Z' ist $-COOCH_2C_6H_5$, * bedeutet überwiegend S- oder überwiegend R-Konfiguration, wobei der Rest R im Amin $RNH_2$ kein oder mindestens ein chirales C-Atom enthält):

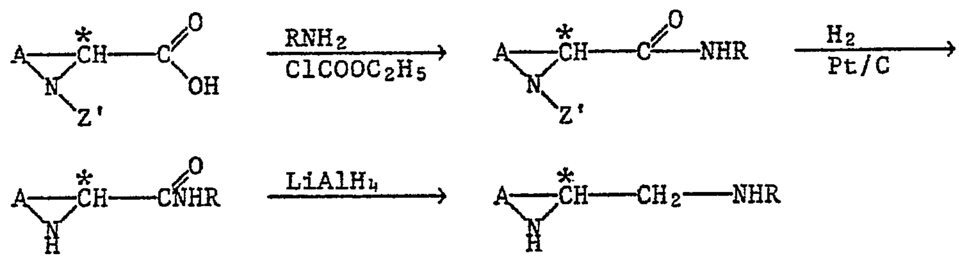


2-Piperidin-, 2-Pyrrolidin- und 2-Indolincarbonsäure und deren Stereoisomere sind bekannt. Geeignete Amine $R-NH_2$ sind z.B. Anilin, 1-Amino-3-methylbenzol, 1-Amino-2,6-dimethylbenzol, α-Naphthylamin, (R)-1-Phenylethylamin, (S)-1-Phenylethylamin, (R)-1,2-Diphenylethylamin.

Ein weiterer Gegenstand der Erfindung ist die Verwendung der erfindungsgemässen Iridiumkomplexe als enantioselektive homogene Katalysatoren insbesondere für die Transferhydrierung prochiraler Ketone mit sekundären Alkoholen. Als sekundärer Alkohol ist besonders Isopropanol geeignet. Die Reaktion wird vorteilhaft unter Sauerstoffausschluss bei erhöhter Temperatur, z.B. 40 - 120°C, durchgeführt. Zweckmässig wird der verwendete sekundäre Alkohol als Lösungsmittel verwendet. Die Katalysatormenge beträgt bevorzugt $10^{-2}$ bis $10^{-5}$ Mol/l, bezogen auf das Reaktionsvolumen. Die Reaktion wird bevorzugt in Gegenwart einer Base, besonders NaOH, durchgeführt.

Die nachfolgenden Beispiele erläutern die Erfindung näher. Die Bestimmung des Enantiomerenüberschusses (ee) erfolgt nach Mosher [J. Org. Chem 34, S. 2543 (1969)].

<u>Beispiele 1 - 9:</u> Unter Argonschutzgas werden 0,469 g (1.0 mmol) Bis-(acetonitril) (cycloocta-1,5-dien)iridium-tetrafluoroborat in 15 ml Dichlormethan gelöst. Bei Raumtemperatur und unter Rühren wird eine Lösung aus 5 ml Dichlormethan und 1.0 mmol des gemäss Tabelle 1 N-substituierten 2-(Aminomethyl)-6-methyl-pyridins (N,N-Ligand) zugetropft. Nach 1 Stunde wird das Reaktionsgemisch bei ca. 600 Pa auf ca. ein Drittel des Volumens eingeengt. Erfolgt keine spontane Kristallisation des Produktes, werden 60 ml Diethylether zugegeben, wobei das Produkt innerhalb 3 Stunden als fester Niederschlag anfällt. Das feinpulvrige Produkt wird unter Argon abgesaugt, dreimal mit Diethylether gewaschen und ca. 16 Stunden bei 0.1 Pa getrocknet. Die Ausbeuten betragen 80 % d. Theorie. Farbe sowie Elementaranalyse der erhaltenen Komplexe sind in Tabelle 1 aufgeführt.

Tabelle 1

| Beispiel Nr. | Komplex | N,N = 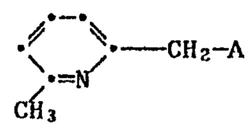 A (absolute Konfiguration) | Farbe | Elementaranalyse |
|---|---|---|---|---|
| 1 | [Ir(N,N)(COD)]$BF_4$ | 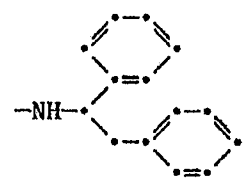 (R) | gelb | Gef: C 49,5; H 5,02; N 4,10; Ir 27,5<br>Ber: C 50,51; H 4,97; N 4,06; Ir 27,87 |
| 2 | " | 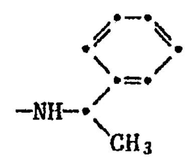 (S) | orange | Gef: C 44,23; H 5,27; N 4,37; Ir 28,5<br>Ber: C 44,38; H 5,02; N 4,50; Ir 30,87 |
| 3 | " | 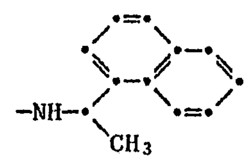 (R) | orangerot | Gef: C 46,33; H 4,96; N 4,01; Ir 27,2<br>Ber: C 46,35; H 5,19; N 4,00; Ir 27,47 |
| 4 | " | 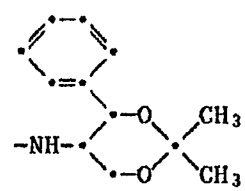 (4S,5S) | dunkel-gelb | Gef: C 43.02; H 4,89; N 3,76; Ir 25,4<br>Ber: C 43,03; H 5,62; N 3,72; Ir 25,5 |

Tabelle 1, Fortsetzung

| Beispiel Nr. | Komplex | N,N = 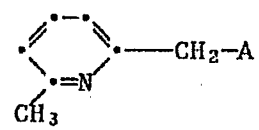 A (absolute Konfiguration) | Farbe | Elementaranalyse [Gew. %] |
|---|---|---|---|---|
| 5 | $[Ir(N,N)(COD)]BF_4$ | 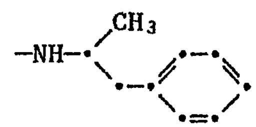 (S) | gelb | Gef: C 44,56; H 5,11; N 4,64; Ir 28,7<br>Ber: C 44,45; H 5,37; N 4,15; Ir 28,45 |
| 6 | " | 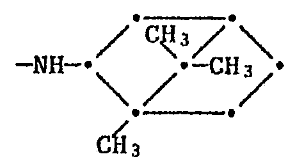 (1R) | hellgelb | Gef: C 44,73; H 5,89; N 4,55; Ir 27,7<br>Ber: C 44,05; H 6,21; N 4,11; Ir 28,2 |
| 7 | " | 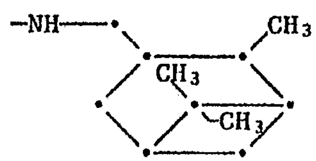 (1S, 2S, 3S, 5R) | hellbeige | Gef: C 46,11; H 6,01; N 4,32<br>Ber: C 46,08; H 6,25; N 4,13 |

Tabelle 1, Fortsetzung

| Beispiel Nr. | Komplex | N,N = 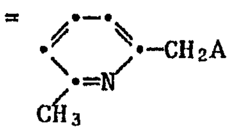 A (absolute Konfiguration) | Farbe | Elementaranalyse [Gew. %] |
|---|---|---|---|---|
| 8 | [Ir(N,N)(COD)]$BF_4$ | 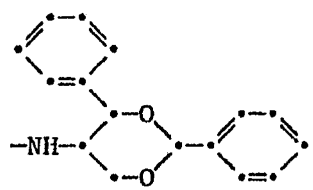 (2R, 4S, 5S) | gelbgrün | Gef: C 48,15; H 4,98; N 3,55; Ir 24,9 Ber: C 48,64; H 4,88; N 3,66; Ir 25,1 |
| 9 | " | 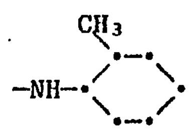 (1S, 2R) | gelb | Gef: C 42,20; H 5,56; N 4,52; Ir 29,6 Ber: C 42,38; H 5,82; N 4,44; Ir 30,82 |

COD ist Cycloocta-1,5-dien

Beispiele 10 - 19: Analog der Beispiele 1 - 9 werden die in Tabelle 2 näher beschriebenen N-substituierten 2-(Aminomethyl)pyridine mit Bis(acetonitril) (cycloocta-1,5-dien)iridium-tetra-fluoroborat zur Reaktion gebracht. Nach analoger Aufarbeitung werden die in Tabelle 2 aufgeführten Komplexe erhalten.

Tabelle 2

| Beispiel Nr. | Komplex | N,N = 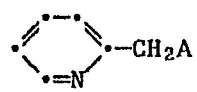 A (absolute Konfiguration) | Farbe | Elementaranalyse [Gew. %] |
|---|---|---|---|---|
| 10 | [Ir(N,N)(COD)]$BF_4$ | 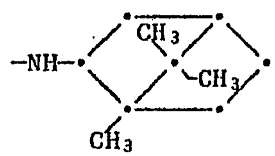 (1R) | gelblich-beige | Gef: C 44,65; H 5,85; N 4,29; Ir 28,5<br>Ber: C 44,38; H 5,90; N 4,31; Ir 29,5 |
| 11 | " | 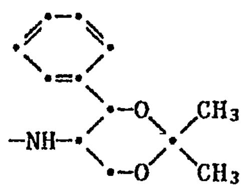 (4S,5S) | gelblich-beige | Gef: C 49,16; H 4,89; N 4,10; Ir 26,2; F 10,27<br>Ber: C 49,18; H 4,54; N 3,82; Ir 26,24; F 10,37 |
| 12 | " | 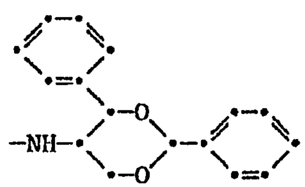 (2R, 4S, 5S) | gelb-orange | Gef: C 45,67; H 4,66; N 4,02; Ir 26,0<br>Ber: C 45,62; H 4,86; N 4,09; Ir 28,08 |
| 13 | " | 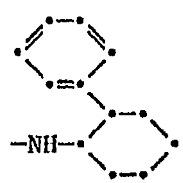 (1S, 2S) | gelb | Gef: C 45,92; H 5,37; N 4,37<br>Ber: C 45,79; H 5,44; N 4,45 |

Tabelle 2, Fortsetzung

| Beispiel Nr. | Komplex | N,N = 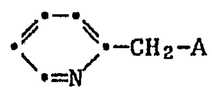 A (absolute Konfiguration) | Farbe | Elementaranalyse [Gew. %] |
|---|---|---|---|---|
| 14 | [Ir(N,N)(COD)]$BF_4$ | 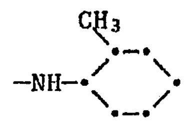 (1S, 2R) | orange | Gef.: C 42,1; H 5,5; N 4,8; Ir 31,4<br>Ber.: C 41,45; H 5,47; N 4,6; Ir 31,59 |
| 15 | " | 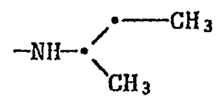 (R) | beige | Gef.: C 37,92; H 5,23; N 4,73; Ir 30,6<br>Ber.: C 37,94; H 5,70; N 4,66; Ir 31,9 |
| 16 | " | 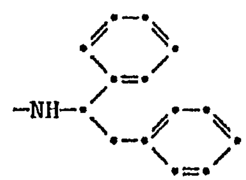 (R) | orange | Gef.: C 49,24; H 4,72; N 4,26; Ir 28,2<br>Ber.: C 49,78; H 4,77; N 4,15; Ir 28,45 |
| 17 | " | 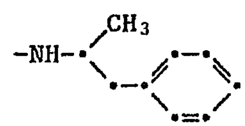 (S) | gelb-orange | Gef.: C 44,61; H 4,78; N 4,47; Ir 30,1<br>Ber.: C 45,03; H 4,93; N 4,57; Ir 3,13 |

Tabelle 2, Fortsetzung

| Beispiel Nr. | Komplex | N,N = 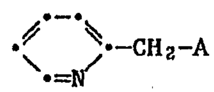 A (absolute Konfiguration) | Farbe | Elementaranalyse [Gew. %] |
|---|---|---|---|---|
| 18 | [Ir(N,N)(COD)]$BF_4$ | 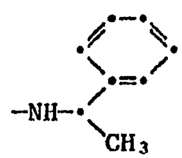 (R) | orange | Gef.: C 43,66; H 4,88; N 4,52; Ir 30,5<br>Ber.: C 43,43; H 4,80; N 4,60; Ir 31,5 |
| 19 | " | 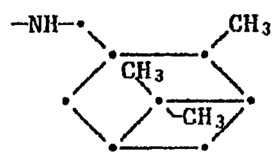 (1S, 2S, 3S, 5R) | orange | Gef.: C 45,40; H 5,97; N 4,16; Ir 29,1<br>Ber.: C 45,25; H 6,08; N 4,22; Ir 29,0 |

Beispiele 20 - 26: Analog der Beispiele 1 - 9 werden die in Tabelle 3 näher beschriebenen N-substituierten 2-(Aminomethyl)piperidine mit Bis(acetonitril)(cycloocta-1,5-dien)iridium-tetrafluoroborat zur Reaktion gebraucht. Nach analoger Aufarbeitung werden die in Tabelle 3 aufgeführten Komplexe erhalten.

Beispiele 27 - 30: Analog der Beispiele 1 - 9 werden die in Tabelle 4 aufgeführten N-substituierten 2-(Aminomethyl)pyrrolidine mit Bis(acetonitril)(cycloocta-1,5-dien)iridium-tetra-fluoroborat zur Reaktion gebracht. Nach analoger Aufarbeitung werden die in Tabelle 4 aufgeführten Komplexe erhalten.

Beispiele 31 - 33: Analog der Beispiele 1 - 9 werden die in Tabelle 5 aufgeführten N,N-Liganden mit Bis(acetonitril)cycloocta-1,5-dien)iridiumtetrafluoroborat zur Reaktion gebracht. Nach analoger Aufarbeitung werden die in Tabelle 5 aufgeführten Komplexe erhalten.

Tabelle 3

| Beispiel Nr. | Komplex | N,N = 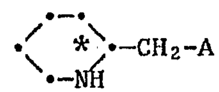 A (absolute Konfiguration) | Farbe | Elementaranalyse [Gew. %] |
|---|---|---|---|---|
| 20 | $[Ir(N,N)(COD)]BF_4$ | 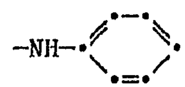 (S) | grün | Gef.: C 40,64; H 5,35; N 4,82; Ir 31,9<br>Ber.: C 40,34; H 5,42; N 4,70; Ir 32,28 |
| 21 | " | 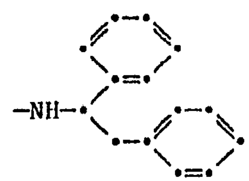 (R,R) | beige | Gef.: C 46,80; H 5,57; N 3,98; Ir 26,6<br>Ber.: C 46,86; H 5,90; N 3,90; Ir 26,8 |
| 22 | " | 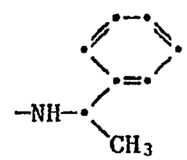 (R,R) | hell-beige | Gef.: C 42,82; H 5,53; N 4,56; Ir 31,1<br>Ber.: C 42,38; H 5,82; N 4,49; Ir 30,82 |
| 23 | " | 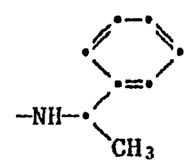 (S,R) | gelb | Gef.: C 41,56; H 5,64; N 4,43; F 11,74<br>Ber.: C 41,19; H 5,97; N 4,37; F 11,85 |

Tabelle 3, Fortsetzung

| Beispiel Nr. | Komplex | N,N = 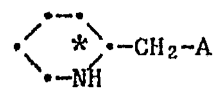 A (absolute Konfiguration)[1] | Farbe | Elementaranalyse [Gew. %] |
|---|---|---|---|---|
| 24 | $[Ir(N,N)(COD)]BF_4$ | 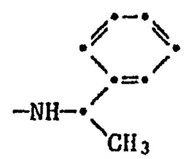 (R,S) (Diastereomerengemisch: R,R: S,R = 43 : 57) | grünlich gelb | Gef.: C 42,68; H 5,43; N 4,64; Ir 30,1 Ber.: C 42,38; H 5,82; N 4,49; Ir 30,82 |
| 25 | " | 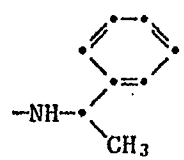 (Diastereomerengemisch R,S: S,S = 42 : 58) | beige | Gef.: C 43,41; H 5,67; N 4,63; Ir 30,1 Ber.: C 43,64; H 5,66; N 4,63; Ir 31,74 |

Tabelle 3, Fortsetzung

| Beispiel Nr. | Komplex | N,N = 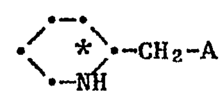 A (absolute Konfiguration) | Farbe | Elementaranalyse [Gew. %] |
|---|---|---|---|---|
| 26 | $[Ir(N,N)(COD)]BF_4$ | 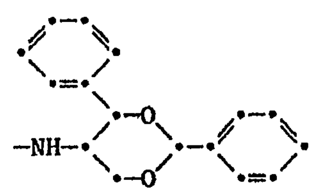 (Diastereomerengemisch: S,4S,5S: R,4S,5S ≈ 1:1) | beige-orange | Gef.: C 46,41; H 5,34; N 3,53; Ir 24,4<br>Ber.: C 46,45; H 5,72; N 3,61; Ir 24,78 |

1) Die erste Bezeichnung (R oder S) bezieht sich auf die 2-Position im Piperidinring.

<u>Tabelle 4:</u>

| Beispiel Nr. | Komplex | N,N = 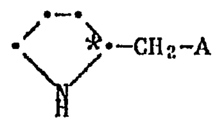 A (absolute Konfiguration)[1] | Farbe | Elementaranalyse [Gew. %] |
|---|---|---|---|---|
| 27 | [Ir(N,N)(COD)]$BF_4$ | 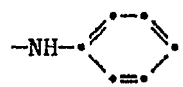 (S) | gelb | Gef.: C 39,46; H 5,14; N 4,79; Ir 33,1<br>Ber.: C 39,25; H 5,20; N 4,82; Ir 33,05 |
| 28 | " | 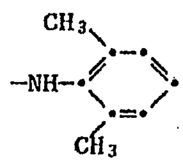 (S) | zitronen-gelb | Gef.: C 42,52; H 5,50; N 4,66; Ir 32,2<br>Ber.: C 42,64; H 5,45; N 4,74; Ir 32,50 |
| 29 | " | 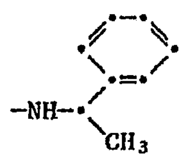 (S,S) | blassgelb | Gef.: C 40,84; H 5,42; N 4,75; Ir 31,5<br>Ber.; C 41,38; H 5,62; N 4,60; Ir 31,53 |
| 30 | " | 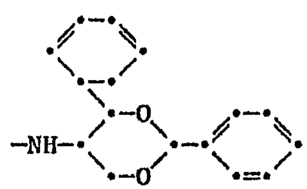 (S, 2R, 4S, 5S) | gelb | Gef.: C 46,36; H 5,11; N 3,90; Ir 25,2<br>Ber.: C 46,84; H 5,42; N 3,77; Ir 25,85 |

1) Die erste Bezeichnung (R oder S) bezieht sich auf die 2-Position des Pyrrolidinrings

Tabelle 4, Fortsetzung

| Beispiel Nr. | Komplex | N,N-Ligand | Farbe | Elementaranalyse |
|---|---|---|---|---|
| 31 | $[Ir(N,N)(COD)]BF_4$ | 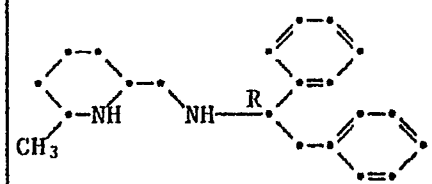<br>Diastereomerengemisch | braun | Gef.: C 49,13; H 5,96; N 3,94; Ir 25,8<br>Ber.: C 48,81; H 5,93; N 3,93; Ir 26,0 |
| 32 | " | 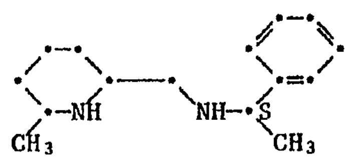<br>Diastereomerengemisch | gelb | Gef.: C 43,80; H 5,65; N 4,51; Ir 29,4<br>Ber.: C 43,54; H 5,56; N 4,42; Ir 30,29 |
| 33 | " | 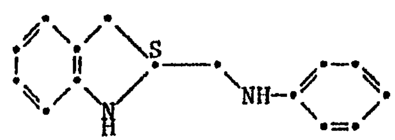 | grünlich gelb | Gef.: C 44,27; H 4,90; N 4,44; Ir 29,7<br>Ber.: C 43,88; H 4,80; N 4,45; Ir 30,53 |

Beispiel 34:

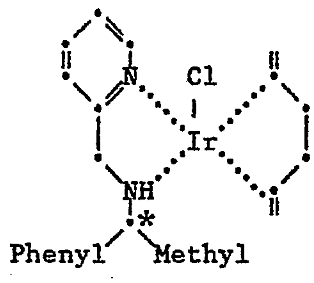

Unter Argon-Schutzgas werden 0,337 (0,376 mMol) Di-μ-chlorotetrakis-(cycloocten)diiridium(I) in 26 ml Benzol gelöst. Bei 10°C werden 2,6 ml 1,5 Hexadien zugetropft. Nach 1 Stunde Rühren wird eine Lösung des Diamins in 2 ml Benzol zugetropft. Das Reaktionsgemisch wird 1 Stunde bei Raumtemperatur gerührt und danach über Celite filtriert. Das Produkt fällt bei Zugabe von 120 ml Hexan als beiges Pulver aus. Es wird filtriert, mehrmals mit Hexan gewaschen und 18 Stunden bei 0,1 Pa getrocknet.

Farbe: beige

| Mikroanalyse (Formel: $C_{20}H_{26}N_2ClIr$): | C | H | N | Cl |
|---|---|---|---|---|
| Berechnet | 46,01 | 5,02 | 5,37 | 6,79 |
| Gefunden | 40,61 | 4,74 | 4,1 | 7,58 |

Das Produkt enhält ca. 20 % Di-μ-chlorotetrakis(cycloocten)diiridium(I).

Beispiel 35 (Anwendungsbeispiel): 6,42 mg des gemäss Beispiel 2 hergestellten Komplexes werden unter Sauerstoffausschluss (Argonatmosphäre) in 39,5 ml Isopropanol gelöst. Nach 1 Stunde Rühren bei 60°C werden 0,42 ml 0,1 normale Natriumhydroxid-Lösung zugegeben. Es wird eine weitere Stunde bei 60°C gerührt und anschliessend eine Lösung aus 39,5 ml Isopropanol und 1,55 g Butyrophenon unter Sauerstoffauschluss hinzugefügt. Das molar Verhältnis Substrat zu Katalysator beträgt somit [S]/[kat.] = 1000, die Katalysatorkonzentration $1{,}33\cdot10^{-4}$ Mol/l.

Nach 3 Stunden bei 60°C wird gaschromatographisch (OV 101, 120°C, isotherm) eine Ausbeute an 1-Phenyl-1-butanol von 90,9 % der Theorie bestimmt.

Zur Bestimmung des Enantiomerengehalts nach Mosher wird eine Probe des Reaktionsgemisches (ca. 0,5 ml) weitgehend vom Lösungsmittel befreit und bei 0°C mit 50 μl optisch reinem α-Methoxy-α-trifluormethylphenylacetylchlorid sowie 0,25 ml trockenem Pyridin versetzt. Nach 15 Minuten wird 30 Minuten auf 70°C erwärmt. nach Abkühlen mit 3 ml 10 prozentiger Citronensäurelösung versetzt und mit Ether die diasteromeren Ester extrahiert.

Gaschromatographisch (Kapillarsäule CW 20, 190°C) wird ein Enantiomerenüberschuss an (S)-1-Phenyl-butanol von ee = 54,2 % bestimmt.

Beispiel 36 (Anwendungsbeispiel): Analog Beispiel 35 wird der Komplex nach Beispiel 2 zur katalytischen, enantioselektiven Transferhydrierung von Isobutyrophenon verwendet.

Nach 1 Stunde 45 Minuten beträgt die Ausbeute an 1-Phenyl-2-methylpropanol 95,3%, der Enantiomerenüberschuss ee = 57,3% (S).

Beispiel 37 (Anwendungsbeispiel): Analog Beispiel 35 wird der Komplex nach Beispiel 1 zur katalytischen, enantioselektiven Transferhydrierung von Butyrophenon verwendet.

Nach 12 Stunden 45 Minuten beträgt die Ausbeute an 1-Phenylbutanol 90,4 %, der Enantiomerenüberschuss ee = 60,4 % (R).

Beispiel 38 (Anwendungsbeispiel): Entsprechend Beispiel 35 wird der Komplex nach Beispiel 12 zur katalytischen enantioselektiven Transferhydrierung von Butyrophenon verwendet. Es werden jedoch die Konzentrationsverhältnisse [S]/[kat.] = 100, [kat.] = $2\cdot10^{-3}$ Mol/l gewählt.

Nach 18 Stunden beträgt die Ausbeute 78,8 % an 1-Phenylbutanol, der Enantiomerenüberschuss beträgt ee = 55,3 % (S).

Beispiel 39 (Anwendungsbeispiel): Entsprechend Beispiel 35 wird der Komplex nach Beispiel 23 zur katalytischen, enantioselektiven Transferhydrierung von Butyrophenon verwendet. Es werden jedoch die Konzentrationsverhältnisse [S]/]kat.] = 1000,
[kat.] = $1{,}33\cdot10^{-4}$ Mol/l gewählt.

Nach 20 Stunden beträgt die Ausbeute 88,8 % an 1-Phenylbutanol, der Enantiomerenüberschuss ee = 60,1 % (R).

Beispiel 40 (Anwendungsbeispiel): Entsprechend Beispiel 35 wird der Komplex nach Beispiel 25 zur katalytischen, enantioselektiven Transferhydrierung von Butyrophenon verwendet. Es werden jedoch die Konzentrationsverhältnisse [S]/[kat.] = 1000,
[kat.] = $1{,}33\cdot10^{-4}$ Mol/l gewählt.

Nach 20 Stunden beträgt die Ausbeute 45,3 % an 1-Phenylbutanol, der Enantiomerenüberschuss ee = 54,1 % (S).

Beispiel 41 (Anwendungsbeispiel): Entsprechend Beispiel 35 wird der Komplex nach Beispiel 20 zur katalytischen, enantioselektiven Transferhydrierung von Butyrophenon verwendet. Es werden jedoch die Konzentrationsverhältnisse [S]/[kat.] = 1000,
[kat.] 1,33•10$^{-1}$ Mol/l gewählt.

Nach 20 Stunden 30 Minuten beträgt die Ausbeute 31,2 % an 1-Phenylbutanol, der Enantiomerenüberschuss ee = 49,3 % (R)

Beispiel 42 (Anwendungsbeispiel): Analog Beispiel 35 wird der Komplex nach Beispiel 27 zur katalytischen, enantioselektiven Transferhydrierung von Butyrophenon verwendet.

Nach 20 Stunden beträgt die Ausbeute 39,0 % an 1-Phenylbutanol, der Enantiomerenüberschuss ee = 29,2 % (R).

Beispiel 43 (Anwendungsbeispiel): Entsprechend Beispiel 35 wird der Komplex nach Beispiel 32 zur katalytischen, enantioselektiven Transferhydrierung von Butyrophenon verwendet. Es werden jedoch die Konzentrationsverhältnisse [S]/[kat] = 1000,
[kat.] = 1,33•10$^{-4}$ Mol/l gewählt.

Nach 2 Stunden beträgt die Ausbeute 95,0 % an 1-Phenylbutanol, der Enantiomerenüberschuss ee = 60,4 % (S).

## Patentansprüche

1. Optisch aktive Iridiumkomplexe der Formel I und Gemische der Diastereomeren,

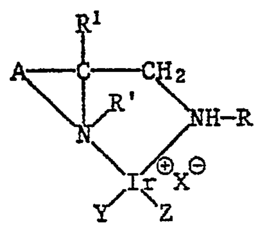 (I),

worin $R^1$ und R′ H bedeuten und A für einen Rest der Formeln II, IIa oder IIb

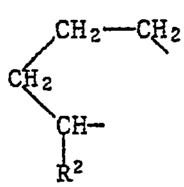 (II), 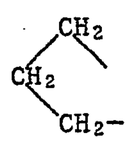 (IIa), 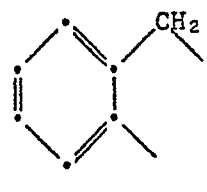 (IIb)

steht, oder $R^1$ und R′ eine Bindung und A einen Rest der Formel IIc

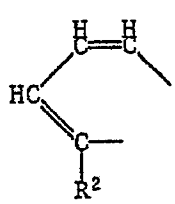 (IIc)

darstellen, in denen $R^2$ -H oder $-CH_3$ ist, $X^\ominus$ das Anion einer anorganischen oder organischen Säure darstellt, Y und Z je Ethylen oder Y und Z zusammen ein offenkettiges oder cyclisches Dien mit 6 - 10 C-Atomen, dessen Diengruppen über ein oder zwei C-Atome verbunden sind, bedeuten; und R ein Kohlenwasserstoffrest mit mindestens einem chiralen C-Atom oder ein mindestens ein Heteroatom enthaltender Kohlenwasserstoffrest mit mindestens einem chiralen C-Atom ist, oder A einen Rest der Formeln II, IIa oder IIb und R Phenyl, Naphthyl, 2-Methylphen-1-yl oder 2,6-Dimethylphen-1-yl bedeuten.

2. Iridiumkomplexe der Formel I gemäss Anspruch 1, worin $X^\ominus$ für $BF_4^\ominus$, $ClO_4^\ominus$, $CF_3SO_3^\ominus$ oder $PF_6^\ominus$ steht.

3. Iridiumkomplexe der Formel I gemäss Anspruch 1, worin Y und Z zusammen 1,5-Cyclooctadien, Norbornadien oder 1,5-Hexadien bedeuten.

4. Iridiumkomplexe der Formel I gemäss Anspruch 1, worin R ein Rest der Formel III

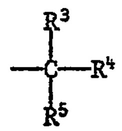

(III)

ist, worin $R^3$, $R^4$ und $R^5$ voneinander verschieden sind, wenn sie nicht mindestens 1 chirales C-Atom enthalten, und für ein Wasserstoffatom, $C_1$-$C_4$-Alkyl, $C_1$-$C_4$-Alkoxy, unsubstituiertes oder durch $C_1$-$C_4$-Alkyl oder Phenyl substituiertes Cycloalkyl mit 5 bis 7 Ring C-Atomen, unsubstituiertes oder durch $C_1$-$C_4$-Alkyl oder Phenyl substituiertes Cycloalkyalkyl mit 5 bis 7 Ring-C-Atomen und 1 oder 2 C-Atomen in der Alkylengruppe, Phenyl, Naphthyl, Benzyl oder β-Phenylethyl sind; oder $R^4$ und $R^5$ zusammen durch $C_1$-$C_4$-Alkyl oder Phenyl substituiertes lineares $C_4$- oder $C_5$-Alkylen, $C_3$- oder $C_4$-Oxaalkylen oder $C_3$-Dioxaalkylen mit mindestens einem chiralen C-Atom bedeuten.

5. Iridiumkomplexe der Formel I gemäss Anspruch 4, worin $R^3$ in Formel III H bedeutet.

6. Iridiumkomplexe der Formel I gemäss Anspruch 4, worin in Formel III $R^3$ H bedeutet, $R^4$ für $C_1$-$C_4$-Alkyl $C_1$-$C_4$-Alkoxy oder Phenyl und $R^5$ für Phenyl, Benzyl oder Naphthyl stehen, wobei $R^3$ und $R^4$ nicht beide Phenyl sind; oder $R^3$ und $R^5$ H bedeuten und $R^4$ der Formel

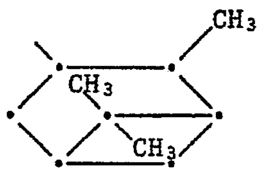

entspricht; oder die Gruppe $-CR^3R^4R^5$ der Formel

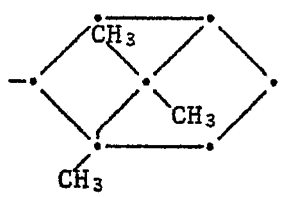

entspricht; oder $R^3$ für H steht $R^4$ und $R^5$ zusammen in 2-Stellung durch $C_1$-$C_4$-Alkyl substituiertes Pentamethylen oder in 1- und/oder 3-Stellung durch $C_1$-$C_4$-Alkyl oder Phenyl substituiertes 2,4-Dioxapentylen bedeuten.

7. Iridiumkomplexe der Formel I gemäss Anspruch 4, worin der Rest der Formel III den Resten

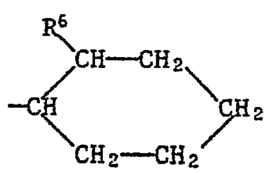

oder

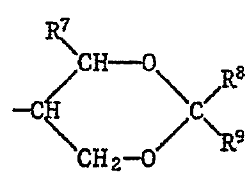

entspricht, worin $R^6$ Methyl oder Phenyl ist, $R^7$ Methyl oder Phenyl darstellen, $R^8$ und $R^9$ Methyl oder $R^8$ H und $R^9$ Phenyl bedeuten.

8. Iridiumkomplexe der Formel I gemäss Anspruch 1, worin $R^1$ und R′ eine Bindung und A ein Rest der Formel IIc ist, in dem $R^2$ für Methyl steht, und R den Resten

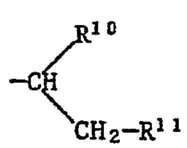

oder

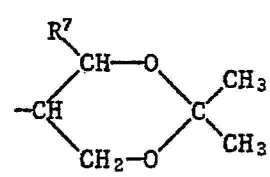

entspricht, worin $R^7$ Phenyl ist, $R^{10}$ Phenyl oder Naphthyl und $R^{11}$ H oder $R^{10}$ und $R^{11}$ Phenyl sind.

9. Iridiumkomplexe der Formel I gemäss Anspruch 1, worin $R^1$ und R′ eine Bindung und A ein Rest der Formel IIc sind, in dem $R^2$ für H steht, und R 2-Methylcyclohex-1-yl, 2-Phenylcyclohex-1-yl,

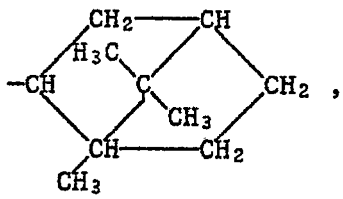 , 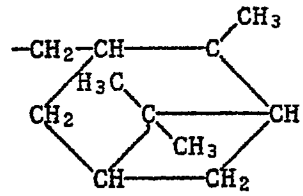

oder

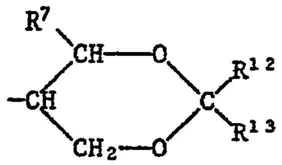 ist,

worin $R^7$ Phenyl und $R^{12}$ und $R^{13}$ $-CH_3$ oder $R^{12}$ H und $R^{13}$ Phenyl bedeuten.

10. Iridiumkomplexe der Formel I gemäss Anspruch 1, worin $R^1$ und $R'$ H und A ein Rest der Formel II sind und R den Rest

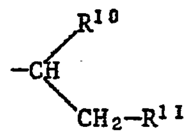

darstellt, worin $R^{10}$ und $R^{11}$ Phenyl, oder $R^{10}$ Phenyl oder Naphthyl und $R^{11}$ H, oder $R^{10}$ Methyl und $R^{11}$ Phenyl bedeuten.

11. Iridiumkomplexe der Formel I gemäss Anspruch 1, worin $R^1$ und $R'$ H und A ein Rest der Formel IIa sind, und R für Phenyl, 2-Methylphen-1-yl oder 2,6-Dimethylphen-1-yl steht.

12. Iridiumkomplexe der Formel I gemäss Anspruch 1, worin $X^\ominus$ für $BF_4^\ominus$ und Y und Z zusammen für 1,5-Cyclooctadien stehen.

13. Verwendung von optisch aktiven Iridiumkomplexen der Formel I gemäss Anspruch 1 als enantioselektive homogene Katalysatoren für die Transferhydrierung von prochiralen Ketonen mit sekundären Alkoholen.

14. Verfahren zur Herstellung von optisch aktiven Iridiumkomplexen der Formel I gemäss Anspruch 1, dadurch gekennzeichnet, dass man $[(\text{Acetonitril})_2(YZ)Ir]^\oplus X^\ominus$ mit einem Amin der Formel IV

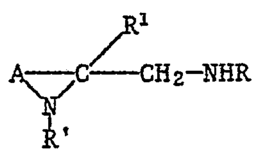 (IV)

umsetzt, worin Y, Z, $X^\ominus$, A, R, $R^1$ und $R'$ die in Anspruch 1 angegebene Bedeutung haben.

**Claims**

1. An optically active iridium complex of the formula I and a mixture of the diastereomers

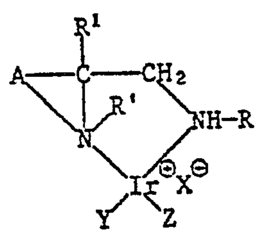 (I)

in which $R^1$ and $R'$ are H and A is a radical of the formula II, IIa or IIb

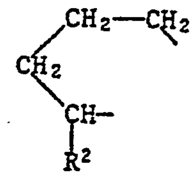 (II), 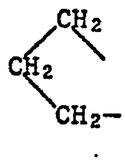 (IIa), 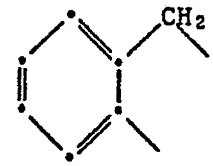 (IIb)

or $R^1$ and $R'$ represent a bond and A is a radical of the formula IIc

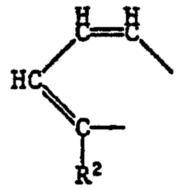 (IIc)

in which $R^2$ is $-H$ or $-CH_3$, $X^\ominus$ is an anion of an inorganic or organic acid, Y and Z are each ethylene, or Y and Z together represent an openchain or cyclic diene having 6–10 C atoms, whose diene groups are bonded via one or two C atoms; and R is a hydrocarbon radical having at least one chiral C atom or a hydrocarbon radical having at least one hetero atom and at least one chiral C atom, or A is a radical of the formula II, IIa or IIb and R is phenyl, naphthyl, 2-methylphen-1-yl or 2,6-dimethylphen-1-yl.

2. An iridium complex of the formula I according to claim 1, wherein $X^\ominus$ is $BF_4^\ominus$, $ClO_4^\ominus$, $CF_3SO_3^\ominus$ or $PF_6^\ominus$.

3. An iridium complex of the formula I according to claim 1, wherein Y and Z together are 1,5-cyclooctadiene, norbornadiene or 1,5-hexadiene.

4. An iridium complex of the formula 1 according to claim 1, wherein R is a radical of the formula III

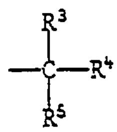 (III)

in which $R^3$, $R^4$ and $R^5$ differ from one another when they do not contain at least 1 chiral C atom, and are a hydrogen atom, $C_1$–$C_4$alkyl, $C_1$–$C_4$alkoxy, cycloalkyl having 5 to 7 ring C atoms which is unsubstituted or substituted by $C_1$–$C_4$alkyl or phenyl, cycloalkylalkyl which is unsubstituted or substituted by $C_1$–$C_4$alkyl or phenyl and has 5 to 7 ring C atoms and 1 or 2 C atoms in the alkylene group, phenyl, naphthyl, benzyl or β-phenylethyl; or $R^4$ and $R^5$ together are $C_1$–$C_4$alkyl-substituted or phenyl-substituted linear $C_4$- or $C_5$-alkylene, $C_3$- or $C_4$-oxaalkylene or $C_3$-dioxaalkylene having at least one chiral C atom.

5. An iridium complex of the formula I according to claim 4, wherein $R^3$ in formula III is H.

6. An iridium complex of the formula I according to claim 4, wherein, in formula III, $R^3$ is H, $R^4$ is $C_1$–$C_4$alkyl, $C_1$–$C_4$alkoxy or phenyl and $R^5$ is phenyl, benzyl or naphthyl, and $R^3$ and $R^4$ are not both phenyl; or $R^3$ and $R^5$ are each H and $R^4$ corresponds to the formula

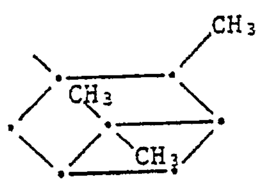

or the group $-CR^3R^4R^5$ corresponds to the formula

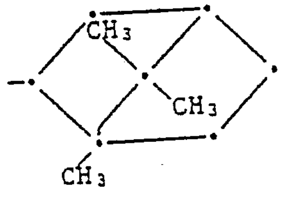

or $R^3$ is H and $R^4$ and $R^5$ together are pentamethylene which is substituted in the 2-position by $C_1$–$C_4$alkyl, or are 2,4-dioxapentylene which is substituted in the 1- and/or 3-position by $C_1$–$C_4$alkyl or phenyl.

7. An iridium complex of the formula I according to claim 4, wherein the radical of the formula III corresponds to the radicals

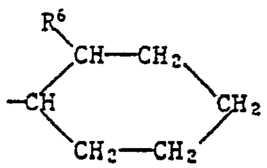

or

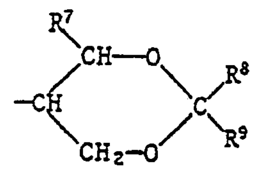

in which $R^6$ is methyl or phenyl, $R^7$ is methyl or phenyl and $R^8$ and $R^9$ are methyl, or $R^8$ is H and $R^9$ is phenyl.

8. An iridium complex of the formula I according to claim 1, wherein $R^1$ and $R'$ are a bond and A is a radical of the formula IIc, in which $R^2$ is methyl and R corresponds to the radicals

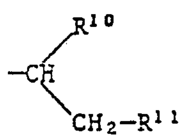

or

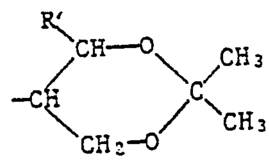

in which $R^7$ is phenyl, $R^{10}$ is phenyl or naphthyl and $R^{11}$ is H, or $R^{10}$ and $R^{11}$ are phenyl.

9. An iridium complex of the formula I according to claim 1, wherein $R^1$ and $R'$ are a bond and A is a radical of the formula IIc, in which $R^2$ is H and R is 2-methylcyclohex-1-yl, 2-phenylcyclohex-1-yl

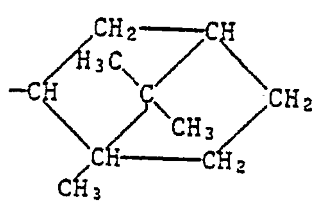

,

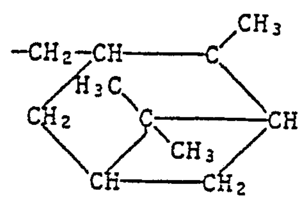

or

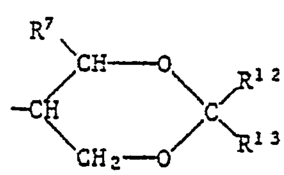

in which $R^7$ is phenyl and $R^{12}$ and $R^{13}$ are $-CH_3$, or $R^{12}$ is H and $R^{13}$ is phenyl.

10. An iridium complex of the formula I according to claim 1, wherein $R^1$ and $R'$ are H and A is a radical of the formula II and R is a radical

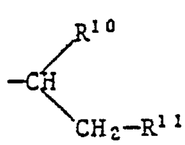

in which $R^{10}$ and $R^{11}$ are phenyl, or $R^{10}$ is phenyl or naphthyl and $R^{11}$ is H, or $R^{10}$ is methyl and $R^{11}$ is phenyl.

11. An iridium complex of the formula I according to claim 1, wherein $R^1$ and $R'$ are H and A is a radical of the formula IIa, and R is phenyl, 2-methylphen-1-yl or 2,6-dimethylphen-1-yl.

12. An iridium complex of the formula I according to claim 1, wherein $X^{\ominus}$ is $BF_4^{\ominus}$ and Y and Z together are 1,5-cyclooctadiene.

13. Use of an optically active iridium complex of the formula I according to claim 1 as an enantioselective homogeneous catalyst for the transfer hydrogenation of prochiral ketones with secondary alcohols.

14. A process for the preparation of an optically active iridium complex of the formula I according to claim 1, wherein $[(acetonitrile)_2(YZ)Ir]^{\oplus}X^{\ominus}$ is reacted with an amine of the formula IV

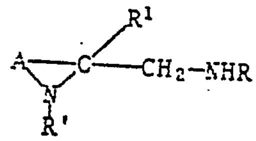

(IV)

in which Y, Z, $X^{\ominus}$, A, R, $R^1$ and R′ have the meaning stated in claim 1.

**Revendications**

1. Complexes d'iridium, optiquement actifs, de formule I et mélange des diastéréomères

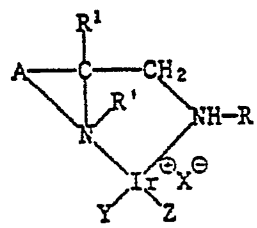

(I),

où $R^1$ et R′ représentent H et A représente un reste répondant aux formules II, IIa ou IIb

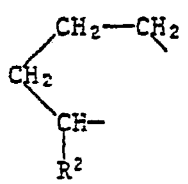

(II),

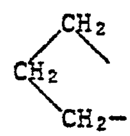

(IIa),

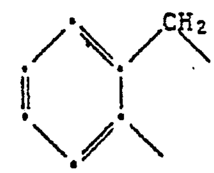

(IIb)

ou bien $R^1$ et R′ forment une liaison et A représente un reste de formule IIc

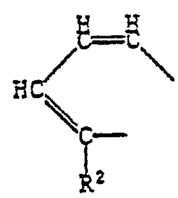

(IIc)

formules dans lesquelles $R^2$ représente $-H$ ou $-CH_3$, $X^{\ominus}$ représente l'anion d'un acide minéral ou organique, Y et Z représentent chacun un groupe éthylène ou bien Y et Z forment ensemble un diène, en chaîne ouverte ou cyclique, ayant 6 à 10 atomes de carbone, dont les groupes diéniques sont reliés par l'intermédiaire d'un ou deux atomes de carbone; et R représente un reste d'hydrocarbure comportant au moins un atome de C chiral ou un reste d'hydrocarbure contenant au moins un hétéro-atome et comportant au moins un atome de C chiral, ou bien A représente un reste répondant aux formules II, IIa ou IIb et R représente un groupe phényle, naphtyle, méthyl-2 phényle-1 ou diméthyl-2,6 phényle-1.

2. Complexes d'iridium de formule I selon la revendication 1, dans lesquels $X^{\ominus}$ représente $BF_4^{\ominus}$, $ClO_4^{\ominus}$, $CF_3SO_3^{\ominus}$ ou $PF_6^{\ominus}$.

3. Complexes d'iridium de formule I selon la revendication 1, dans lesquels Y et Z forment ensemble un groupe cyclooctadiène-1,5, norbornadiène ou hexadiène-1,5.

4. Complexes d'iridium de formule I selon la revendication 1, dans lesquels R représente un reste de formule III

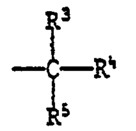

(III)

où $R^3$, $R^4$ et $R^5$ diffèrent les uns des autres quand ils ne contiennent pas au moins un atome de C chiral et représentent chacun un atome d'hydrogène, un groupe alkyle en $C_1$ à $C_4$, alcoxy en $C_1$ à $C_4$, cycloalkyle ayant 5 à 7 atomes de carbone de noyau, non substitués ou substitués par un groupe alkyle ayant 1 à 4 atomes de carbone ou par un groupe phényle, un groupe cycloalkylalkyle ayant 5 à 7 atomes de carbone de noyau et un ou deux atomes de carbone dans le groupe alkylène, non substitués ou substitués par un groupe alkyle en $C_1$ à $C_4$ par un groupe phényle, un groupe phényle, naphtyle, benzyle ou phényl-2 éthyle; ou bien $R^4$ et $R^5$ forment ensemble un groupe alkylène linéaire en $C_4$ ou en $C_5$, oxaalkylène en $C_3$ ou $C_4$ ou dioxaalkylène en $C_3$, comportant au moins un atome de C chiral et substitué par un groupe alkyle en $C_1$ ou $C_4$ ou par un groupe phényle.

5. Complexes d'iridium de formule I selon la revendication 4, dans lesquels $R^3$ représente H dans la formule III.

6. Complexes d'iridium de formule I selon la revendication 4, dans lesquels, dans la formule III, $R^3$ représente H, $R^4$ représente un groupe alkyle en $C_1$ à $C_4$, alcoxy en $C_1$ à $C_4$ ou phényle et $R^5$ représente un groupe phényle, benzyle ou naphtyle, $R^3$ et $R^4$ ne pouvant pas simultanément représenter chacun un groupe phényle; ou bien $R^3$ et $R^5$ représentent H, et $R^4$ répond à la formule

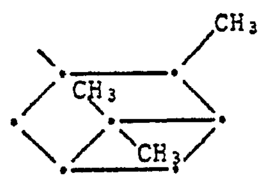

ou bien le groupe $-CR^3R^4R^5$ répond à la formule

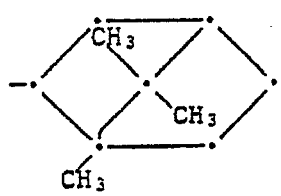

ou bien $R^3$ représente H et $R^4$ et $R^5$ forment ensemble un groupe pentaméthylène, substitué en position 2 par un groupe alkyle en $C_1$ à $C_4$, ou un groupe dioxa-2,4 pentaméthylène, substitué en position 1 et/ou 3 par un groupe alkyle en $C_1$ à $C_4$ ou par un groupe phényle.

7. Complexes d'iridium de formule I selon la revendication 4, dans lesquels le reste de formule III représente les restes

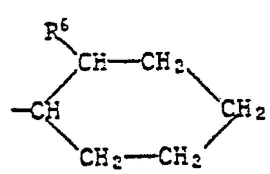

ou

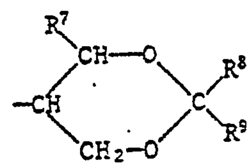

dans lesquels $R^6$ représente un groupe méthyle ou phényle, $R^7$ représente un groupe méthyle ou phényle, $R^8$ et $R^9$ représentent chacun un groupe méthyle ou bien $R^8$ représente H et $R^9$ représente un groupe phényle.

8. Complexes d'iridium de formule I selon la revendication 1, dans lesquels $R^1$ et $R'$ forment une liaison et A représente un reste de formule IIc, dans lequel $R^2$ représente un groupe méthyle, et R représente les restes

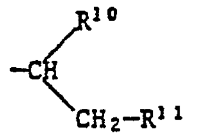

ou

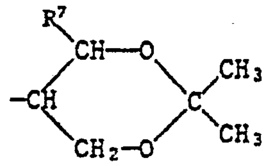

où $R^7$ représente un groupe phényle, $R^{10}$ représente un groupe phényle ou naphtyle et $R^{11}$ représente H ou bien $R^{10}$ et $R^{11}$ représentent chacun un groupe phényle.

9. Complexes d'iridium de formule I selon la revendication 1, dans lesquels $R^1$ et R′ forment une liaison et A représente un reste de formule IIc, dans lequel $R^2$ représente H, et R représente un groupe méthyl-2 cyclohexyle-1, phényl-2 cyclohexyle-1,

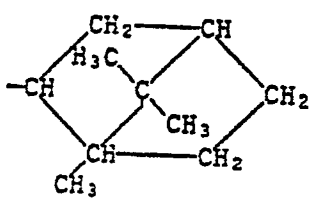

,

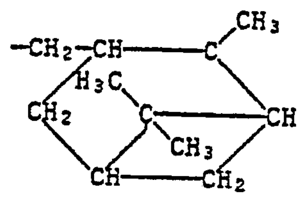

ou

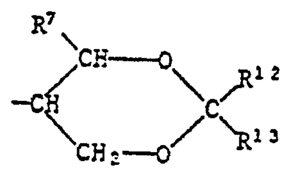

où $R^7$ représente un groupe phényle et $R^{12}$ et $R^{13}$ représentent chacun $-CH_3$, ou bien $R^{12}$ représente H et $R^{13}$ représente un groupe phényle.

10. Complexes d'iridium de formule I selon la revendication 1, dans lesquels $R^1$ et R′ représentent H, et A est un reste de formule II, cependant que R représente le reste

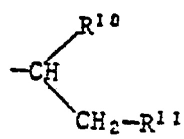

dans laquelle $R^{10}$ et $R^{11}$ représentent chacun un groupe phényle, ou bien $R^{10}$ représente un groupe phényle ou naphtyle et $R^{11}$ représente H, ou bien $R^{10}$ représente un groupe méthyle et $R^{11}$ représente un groupe phényle.

11. Complexes d'iridium de formule I selon la revendication 1, dans lesquels $R^1$ et R′ représentent H et A est un reste de formule IIa, et R représente un groupe phényle, méthyl-2 phényle-1 ou diméthyl-2,6 phényle-1.

12. Complexes d'iridium de formule I selon la revendication 1, dans lesquels $X^{\ominus}$ représente $BF_4$ et Y et Z forment ensemble un groupe cyclooctadiène-1,5.

13. Utilisation de complexes d'iridium, optiquement actifs, de formule I selon la revendication 1, comme catalyseurs homogènes énantio-sélectifs pour l'hydrogénation de transfert de cétones prochirales avec des alcools secondaires.

14. Procédé pour préparer des complexes d'iridium optiquement actifs, de formule I selon la revendication 1, procédé caractérisé en ce qu'on fait réagir $[(\text{acétonitrile})_2(YZ)Ir]^{\oplus}X^{\ominus}$ avec une amine de formule IV

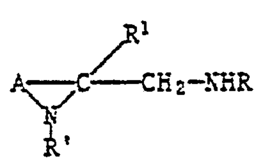

(IV)

où Y, Z, $X^{\ominus}$, A, R, $R^1$ et R′ ont le sens indiqué à la revendication 1.